# EUROPEAN PATENT APPLICATION

(11) **EP 4 775 981 A1**
(43) Date of publication of application: **15.07.2026**
(21) Application number: 25151157.2
(22) Date of filing: 10.01.2025
(51) Int. Cl.: G01N 33/00, G01N 1/22, G01N 1/40, G01M 3/00

(54) **GAS DETECTION SYSTEM AND METHOD FOR MODULATION OF A TARGET GAS CONCENTRATION**

(71) Applicant: Inficon GmbH, 50968 Köln (DE)
(72) Inventor: HELLGREN, Johan, 50968 Köln (DE)
(74) Representative: dompatent

(57) **Abstract**

Gas detection system for modulation of a target gas concentration, the gas detection system comprising a sample gas inlet, a gas sensor sensitive to at least one target gas, and a gas filter adapted to selectively delay the transfer of said target gas over the transfer of at least one other gas component different from the target gas, said gas filter being provided in a gas flow path connecting the sample gas inlet and the gas sensor, characterized in that the gas filter is a gas modulation filter comprising a variable filter time constant for the target gas.

## Description

The invention relates to gas analysis, for example for detecting or monitoring a specific gas.

Gas analysis can be used for gas detection, for detecting gas leaks, or for monitoring the concentration of a gas, for example in a room. A specific gas which is to be detected or monitored is referred to as a target gas, such as refrigerants R134a or R1234yf. However, typical gases used as a target gas for gas detection are also present in air of the test site surroundings. Gas concentrations of such a target gas can therefore only be detected, if they are significantly higher than the natural concentration of said gas component in air. In order to increase the sensitivity of detecting target gas components which are also present in air, gas modulation techniques are used to assess and subtract the natural concentration within air in the test site surroundings. Gas modulation techniques can also be used to measure the concentration of a target gas with a sensor that cannot give an absolute measure of the concentration, but is sensitive to changes in concentration only.

A typical gas modulation set up uses a gas modulation valve which alternatingly connects a sample gas inlet and a reference gas inlet to a gas sensor. Through the sample gas inlet, the actual gas sample taken from the area in which a leak is assumed is introduced. Through the reference gas inlet, a reference gas measurement is taken from another region in which no gas leak is assumed to be present. The valve toggles back and forth between the sample inlet and the reference inlet, and the difference between the concentrations of target gas in both measurements is used as an indication for the presence of a gas leak.

This setup always needs reference gas and cannot be used in applications where a reference gas is not available or when the quality of the reference gas is of disadvantage.

The object of the invention is therefore to provide an improved gas detection system and method for gas modulation.

The gas detection system of the invention is defined by independent claim 1. The method of the invention is defined by independent claim 11.

According to claim 1, the gas detection system comprises a sample gas inlet, a gas sensor sensitive to at least one target gas and, preferably, to variations in target gas concentrations, and a gas filter adapted to selectively delay the transfer of said target gas over the transfer of at least one other gas component. Said gas filter is provided in a gas flow path which connects the sample gas inlet with the gas sensor. A vacuum pump may be provided to generate a pressure difference between the atmosphere and the sample gas inlet to draw in gas through the sample gas inlet, and/or to generate a pressure difference between the sample gas inlet and the gas sensor to convey sampled gas from the sample gas inlet to the gas sensor.

The idea of the invention is to provide the gas filter as a gas modulation filter comprising a variable filter time constant for the target gas. This enables to obtain sample gas measurements at different points in time with different filter time constants for the target gas. If the background level of the target gas is known when comparing the measurements with each other, a measure of the absolute concentration of the target gas can be obtained, even if the sensor is not able to provide an absolute measure of the concentration. When the target gas filter time constant increases, more target gas is adsorbed or delayed by the gas modulation filter. When the target gas filter time constant decreases, previously adsorbed target gas is released from the filter and the delay of the filter is reduced for the target gas.

Generally, an increased target gas filter time constant results in an increased transfer time that the target gas needs to transfer through the filter. A reduced target gas filter time constant results in a reduced transfer time for the transfer of the target gas through the filter. Such filters are also referred to as "selective transfer filters" because different transfer times for selected gas types or gas components are achieved as compared to the transfer times of other gas components. Depending on the type of filter, this can be achieved via a different time behavior of the filter for adsorption and desorption of respective gases. Such filters may also be named "retention filter" or "physical low-pass filter".

The filter time constant may be varied by changing adsorption properties of the filter for adsorbing target gas, by changing accumulation properties of the filter for accumulating target gas, and/or by changing delay properties of the filter for delaying or retarding target gas when travelling through the filter.

The gas modulation filter may be provided with a filter material which changes the filter time constant for the target gas when being heated or when its temperature changes, for example due to temperature dependent adsorption properties for the target gas. The filter material of the gas modulation filter may comprise, consist of and/or be composed of carbon, silica gel and/or molecular sieve. Preferably, the gas modulation filter is adapted to alternatingly accumulate target gas and decumulate target gas, such as by adsorption and desorption or different delay times for the target gas. A modulation control device may be part of or connected with the gas modulation filter in order to control the variation of the target gas filter time constant. The modulation control device may comprise respective electronics, such as a microprocessor or computer adapted or programmed to control the variation of the target gas filter time constant, such as by alternating accumulation and decumulation of the target gas.

The modulation control device may be adapted to vary the target gas filter time constant in a repeated periodic manner, each period having an adsorption time period during which the target gas filter time constant is increased, and a desorption time period during which the target gas filter time constant is decreased. In particular, the target gas filter time constant may be higher than the filter time constant of said other gas components during the adsorption time period, while the target gas filter time constant may be lower than the filter time constant of the other gas components during the desorption time period.

A heating device may be provided for heating a filter material to vary the target gas filter time constant or the adsorption properties of the filter material. The modulation control device can be adapted to control the temperature generated by the heating device, in order to control variation of the target gas filter time constant through changing the temperature of the filter material.

For example, the heating device may comprise heating wires, such as from or comprising Kanthal, provided through or embedded within a filter material of the gas filter. Alternatively or in addition, the heating device may comprise a heating element within the gas flow path upstream of the gas filter, said heating element adapted to heat the gas flow before entering the filter. For example, the heating element may be a NiCr-coil. As an alternative or in addition, the heating device may comprise or be an IR radiator adapted to emit IR radiation into the filter or filter material.

A second gas filter in the form of a second adsorption filter may be provided downstream of the gas modulation filter to reduce variations of gas components that would otherwise influence the quality of the measurement. Such gas components could be H2O that will occur in regular air, can be modulated by the modulation filter (e.g. charcoal filter with varying temperature), and may be detected by the gas sensor. The second gas filter is preferably also a selective transfer filter having a gas filter time constant which is significantly higher for the gas components to be filtered out than for other gas components.

An initial gas filter in the form of an adsorption filter may be provided upstream of the gas modulation filter to remove gas components that would disturb accumulation of the target gas in the gas modulation filter. For example, H2O can affect the adsorption of other gases in charcoal filters. The initial gas filter is preferably replaceable.

The gas sensor may be a pyroelectric sensor in an IR cell. The target gas is preferably a gas with larger molecules as compared to the size of other molecules in air or compared to the average size of molecules in air. In addition or as an alternative, the target gas may have a higher boiling point as compared to other molecules in air or compared to the average boiling point of molecules in air, and/or the target gas may stick to surfaces.

According to the method of the invention, a sample gas is introduced through the sample gas inlet and conveyed to the gas detector through the gas modulation filter.

The target gas filter time constant is increased to accumulate, adsorb and/or delay target gas by the gas modulation filter, and is decreased to release, desorb and/or accelerate the target gas by the gas modulation filter. Thereby, the concentration of target gas in the gas conveyed to the gas detector is increased and decreased.

The gas sensor is used to measure a first concentration of the target gas at a first point in time, and at least a second concentration of the target gas at a second point in time. The first concentration and the second concentration are compared to assess whether target gas is present in the analyzed gas sample.

Preferably, target gas is considered present in the gas sample if the difference between the first concentration and the second concentration is beyond a threshold value.

The amplitude of the signal measured with the gas sensor can be used as the measure for the target gas concentration and may be analyzed over several periods to attenuate noise, if the modulation timing of the modulation control device is known.

In the following, examples of the invention are described with reference to the figures in which
- Fig. 1: shows a first embodiment,
- Fig. 2: shows a second embodiment, and
- Fig. 3: shows a third embodiment.

All embodiments show a gas detection system 10 comprising a gas sensor 12, a sample pump 14 in the form of a vacuum pump, a sample gas inlet 16 through which sample gas is drawn in by the vacuum pump 14, and a gas flow path 18 connecting the sample gas inlet 16, the vacuum pump 14 and the gas sensor 12.

In all embodiments, a gas modulation filter 20 is provided in a gas flow path 18 between the vacuum pump 14 and a gas sensor 12. A modulation control device 22 is electrically connected to a gas modulation filter 20 in order to control the variation of the filter time constant for the target gas to be detected. The target gas may be, for example, R134a which is also present in air surrounding the sample gas inlet 16. The vacuum pump 14 may alternatively be placed anywhere in the gas flow path, such as between the gas modulation filter 20 and the gas sensor 12 or behind the gas sensor 12.

The gas modulation filter 20 comprises a filter material in the form of activated carbon, such as a disc of activated carbon. The adsorption and desorption properties of activated carbon change for specific gas components with changes in temperature of the carbon. Therefore, the adsorption properties of carbon filters depend on the temperature of the filter. A carbon filter can be regenerated by heating it. The filter can be heated by running an electrical current through it. This is achieved and controlled by the modulation control device 22. The current should be varying so that the heating power and thus the temperature of the filter material is varying.

One possible heating scheme is to apply a current to the filter material during a part of the period time of the gas modulation and then turn the current off during the rest of the period. The gas modulation filter will then heat up when the current is on, and will cool down, partly by the gas flow through it, during the rest of the period. Thereby, a repeated periodic cycle of increasing and decreasing target gas filter time constant can be achieved.

One measurement is taken with the gas sensor 12 at a first point in time when the current of the modulation control device 22 is on, i.e. when the target gas filter time constant is increased, and a second measurement is taken at a second point in time when the current is off, i.e. when the target gas filter time constant is decreased. The period comprising the increased target gas filter time constant may be referred to as accumulation time period or adsorption time period. The period with the decreased target gas filter time constant may be referred to as decumulation time period or desorption time period.

The difference between the measurement at the first point in time and the measurement of the second point in time is calculated and compared to respective calculated differences from other cycles of the repeated periodic cycle. If the difference increases, this may be taken as an indication that the increase does not originate from the natural concentration of the target gas in surrounding air, but rather from target gas escaping from a leak in an object under test. In this regard, the calculated difference may be compared to a threshold value.

If the modulation timing and the amplitude of the sensor signal are known, the amplitude can be used as the measure and may be analyzed over several periods to attenuate noise.

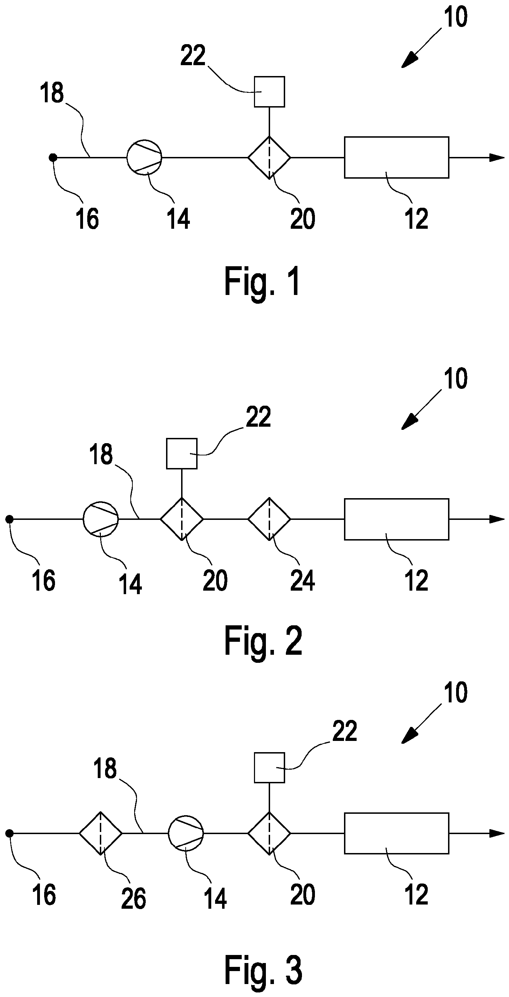

The embodiment of Fig. 2 comprises a second gas filter 24 in the form of an adsorption filter provided in the gas flow path 18 between the gas modulation filter 20 and the gas sensor 12. An advantage of this is that the second adsorption filter 24 can reduce temperature variations and reduce variations in gas concentrations of components that are not of interest. Accordingly, the embodiment of Fig. 2 has two gas filters, the gas modulation filter 20 generating concentration variations for the target gas and possibly other components, and a second gas filter 24 that should reduce the variations of components that otherwise would influence the measurement.

In the embodiment of Fig. 3, an initial adsorption filter 26 is provided in the gas flow path 18 between the sample gas inlet 16 and the vacuum pump 14. The initial gas filter 26 removes gas components that disturb the accumulation of the target gas in the gas modulation filter 20.

The main advantage of the invention is that it can generate gas modulation from a single input flow by the use of a filter with varying accumulation and decumulation properties. A constant flow may be drawn through the sample gas inlet 16 with the vacuum pump 14. This is an advantage over prior art systems using a gas modulation filter which toggles between a sample gas inlet and a reference gas inlet. In such prior art systems, there is no flow from the sample gas inlet when the valve takes gas from the reference gas inlet. The gas sensor may then be blind for exposures to target gas during this period. The invention avoids such blind periods and thereby gives the opportunity to use longer period times which reduces the requirements on the response time of the gas sensor. Therefore, slower gas sensors can be used. The flow of the sample gas through the sample gas inlet 16 may be higher than in prior art systems with gas modulation valves where the flow is divided between the two inputs. Moreover, with a gas modulation filter, the concentration variations can be higher than with gas modulation valves. The target gas is accumulated in a part of the period and then released to the gas flow conveyed through the gas flow path 18 during the rest of the period.

## Claims

1. Gas detection system (10) for modulation of a target gas concentration, the gas detection system (10) comprising a sample gas inlet (16), a gas sensor (12) sensitive to at least one target gas, and a gas filter adapted to selectively delay the transfer of said target gas over the transfer of at least one other gas component different from the target gas, said gas filter being provided in a gas flow path (18) connecting the sample gas inlet (16) and the gas sensor (12),
**characterized in that**
the gas filter is a gas modulation filter (20) comprising a variable filter time constant for the target gas.

2. Gas detection system (10) according to claim 1, wherein the gas filter is provided with a filter material, heating of which changes the target gas filter time constant.

3. Gas detection system (10) according to claim 1 or 2, wherein the gas filter comprises a filter material made of, comprising or consisting of carbon, silica gel and/or molecular sieve.

4. Gas detection system (10) according to one of the previous claims, wherein the gas modulation filter (20) is adapted to alternatingly accumulate target gas and decumulate target gas.

5. Gas detection system (10) according to one of the previous claims, wherein the gas modulation filter (20) comprises or is connected to a modulation control device (22) adapted to control variation of the target gas filter time constant.

6. Gas detection system (10) according to claim 5, wherein the modulation control device (22) is adapted to vary the target gas filter time constant in a repeated periodic cycle, each cycle having an adsorption time period during which the target gas filter time constant is higher than the filter time constant of said other gas component, and a desorption time period during which the target gas filter time constant is lower than the filter time constant of the other gas component.

7. Gas detection system (10) according to claim 5 or 6, wherein the gas modulation filter (20) comprises a heating device for heating a filter material, said modulation control device (22) being adapted to control the temperature generated by the heating device.

8. Gas detection system (10) according to claim 7, wherein the heating device comprises heating wires through a filter material of the filter, a heating element within the gas flow path (18) upstream of the gas filter and adapted to heat the gas flow before entering the filter, and/or an IR radiator adapted to emit infrared (IR) radiation into the filter.

9. Gas detection system (10) according to one of the previous claims, wherein a second adsorption gas filter is provided in the gas flow path (18) downstream of the gas modulation filter (20) to reduce variations of gas components that otherwise would influence the measurement.

10. Gas detection system (10) according to one of the previous claims, wherein an initial adsorption gas filter is provided in the gas flow path (18) upstream of said gas modulation filter (20) to remove gas components that would disturb accumulation of the target gas in the gas modulation filter (20).

11. Method for detection of a target gas using a gas detection system (10) according to one of the previous claims, in which
a sample gas is introduced through the sample gas inlet (16) and conveyed to the gas detector through the gas modulation filter (20),
the target gas filter time constant is increased to accumulate, adsorb and/or delay target gas by the gas modulation filter (20) to thereby increase the concentration of target gas in the gas conveyed to the gas detector, and/or the target gas filter time constant is decreased to release, desorb and/or accelerate the target gas by the gas modulation filter (20) to thereby decrease the concentration of target gas in the gas conveyed to the gas detector,
the gas sensor (12) is used to measure a first concentration of the target gas at a first point in time and a second concentration of the target gas is measured at a second point in time,
the first concentration and the second concentration are compared to assess whether target gas originating from a leak in a device under test is present in the gas sample.

12. Method for detection of a target gas using a gas detection system (10) according to claim 11 wherein target gas is considered to be present in the gas sample if the difference between the first concentration and the second concentration is beyond a threshold value.
